# EUROPEAN PATENT SPECIFICATION

(11) **EP 0 728 489 B1**
(45) Date of publication and mention of the grant of the patent: **23.10.2002**
(21) Application number: 96102511.1
(22) Date of filing: 20.02.1996
(51) Int. Cl.: A61M 1/10, F04B 9/02

(54) **An actuator for pumping elements especially for cardiac assistance devices**
Antrieb für Pumpelemente, insbesondere für Herzunterstützungsvorrichtungen
Actionneur pour éléments de pompe en particulier pour dispositifs d'assistance cardiaque

(30) Priority: 23.02.1995 IT TO950132
(43) Date of publication of application: 28.08.1996
(73) Proprietor: MINISTERO DELL' UNIVERSITA' E DELLA RICERCA SCIENTIFICA E TECNOLOGICA, 00144 Roma (IT)
(72) Inventor: Zagara, Maurizio, 00127 Roma (IT); Ferri, Enrico, 00175 Roma (IT); Mambrito, Bruno, 00044 Frascati (IT); Ronci, Pietro, 06031 Bevagna (Perugia) (IT); Porzi, Carlo, 06034 Foligno (Perugia) (IT)
(74) Representative: Bosotti, Luciano

(56) References cited:
- EP-A- 0 478 499
- US-A- 4 277 706

## Description

The present invention relates to actuators for pumping elements and has been developed with particular attention to its possible use in the production of pumping elements for cardiac assistance devices (artificial ventricles, artificial hearts, etc.). The scope of the invention is not intended to be limited to this specific field however, but extends to the pumping of fluids in general, principally with regard to the pumping of fluids in the biomedical field.

Specifically, the present invention relates to an actuator according to the preamble of claim 1, which is known, e.g. from EP-A-0 478 499.

Also US-A-4 277 706 and US-A-4 493 614 are of some interest for the invention.

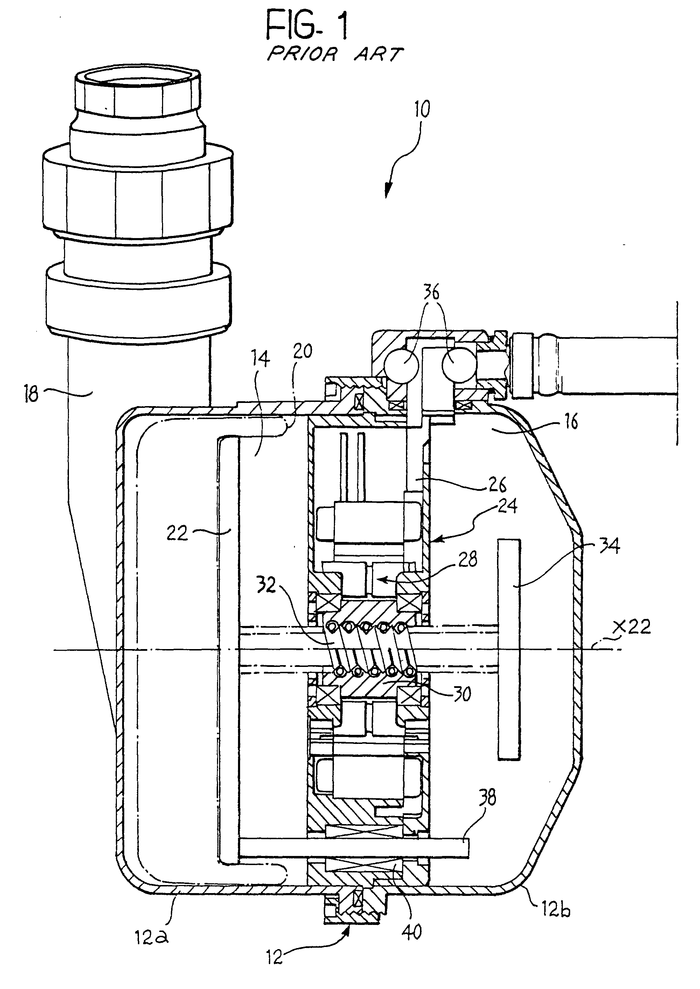

By way of premise and to put the characteristics of the invention better into context, it may be useful to provide a short description of a solution according to the prior art, as illustrated in Figure 1 of the appended drawings.

In this drawing, as an example of a pumping element, a cardiac assistance device (VAD) constituted specifically by a so-called artificial ventricle, is generally indicated 10. The device 10 is housed in a casing or shell 12 of material compatible with the requirements for implantation inside the body (for example, titanium).

Two components or sections can essentially be distinguished within the device 10, that is, a pumping section 14 and an actuating section 16 constituted by an electromechanical actuator which drives the pumping section 14.

The latter is constituted essentially by a portion 12a of the shell 12 having a cup-like shape and having connectors 18 (only one of which is visible in Figure 1, which corresponds to a diametral section of the casing of the device 10, which is of approximately cylindrical shape) for connection to the blood suction or drain and delivery lines. The shell portion 12a houses a flexible bag 20 which is intended, in use, to be connected sealingly to the blood lines connected to the device 10, usually with the interposition of valves (not shown) which regulate the blood flow through the blood lines and the device 10 so as to achieve a pulsed one-way flow. The bag 20 is usually made of a flexible material such as, for example, a polymer having biocompatibility (haemocompatibility) characteristics.

The pumping action takes place as a result of the cyclic compression of the bag 20 brought about by means of a thrust member such as a plate 22 which is movable to and fro along a respective central axis X22.

In practice, the bag 20 is interposed between the base of the cup-shaped portion 12a of the shell 12 and the plate 22. The reciprocating movement of the plate 22 relative to the base of the cup-shaped portion 12a brings about cyclically:
- compression of the bag 20 as a result of the movement of the plate 22 towards the base of the cup-shaped portion 12a, with consequent expulsion of blood from the device 10 through the delivery line, and
- expansion of the bag 20 as a result of the return of the plate 22 to the position in which it is spaced from the base of the cup-shaped portion 12a, consequently permitting the blood to reach the interior of the bag 20 through the intake or drain lines.

The reciprocating movement of the plate 22 along the axis X22 (which usually corresponds to the central axis of the cup-shaped portion 12a) takes place under the action of a motor typically constituted by an electric motor 24. This is usually a brushless motor having an approximately disc-like shape and composed of an annular outer stator 26 fitted in the mouth portion 12 of the shell which encloses the actuating section 16, and a rotor 28. The rotor 28 incorporates or is itself configured in the form of a lead screw 30 which is in a central position (that is, in alignment with the axis X22) and with which a screw 32 is engaged. This is preferably a ball- or roller-bearing screw 32 (the expression "ball-bearing" will be considered below as in any case also including a possible embodiment with the use of rollers) of which the end facing and projecting towards the pumping section 14 is fixed to the plate 22. The other end of the screw 32 projects into the shell portion 12b, however, and preferably carries a further plate 34 having the function of constituting an abutment element which can be brought into abutment with the body of the motor 24 when the screw 32 (and the plate 22 carried thereby) reach a furthest forward position inside the pumping portion 14.

The forward movement of the screw 32 and of the plate 24 as far as the position in which the plate 34 is in abutment with the body of the motor 24 usually takes place only during the first drive stroke of the actuator device. The control unit of the actuator which is usually constituted by an electronic processor device (not shown) connected to the device 10 by means of an electrical connector 36, "learns" the absolute value of the aforementioned furthest forward position, storing it as a spatial reference value which is not usually intended to be reached when the device is in operation. Naturally, this operation takes place by the cyclic rotation of the motor 24 in the two senses with a consequent conversion of the rotary movement of the rotor 28 about the axis X22 into a corresponding axial reciprocating movement of the screw 32 and of the plate 22 along the axis X22.

To prevent the plate 22 from being rotated by the motor 24, the plate has alignment elements such as, for example, one or more rods 38 which extend axially from the plate 22 (usually in positions spaced equiangularly along its periphery), extending through the body of the motor 24 in respective axial cavities 40 in the stator 26.

A motor such as the motor 24 is available commercially and produced by Inland Motors, a division of the Kollmorgen Corporation (Illinois, United States). The structural details of the solution shown in Figure 1 should in any case be considered within the capabilities of a person skilled in the art and, moreover, are not significant *per se* for the purposes of an understanding of the invention.

As stated, a device such as the device 10 is intended preferably for implantation in a patient's body (for example, in an abdominal position). In such a field of use, the restriction of the overall dimensions of the device constitutes a priority requirement, particularly as regards possible implantation in small patients. It should, however, be noted that this requirement may also arise in situations other than those put forward up to now for the use of a pumping element having a structure similar or comparable to that shown in Figure 1.

In particular, with specific reference to Figure 1, it can be seen that the actuation section 16 of the device is occupied only partially (for a portion approximately equal to or slightly less than half of the overall volume) by the motor 24, that is, by the main active element. The rest of the shell portion indicated 12b is occupied by the empty chamber which it is necessary to provide to allow for the reciprocating movement of the screw 32 driven by the motor 24. In order for the screw 32 to be able to bring the plate 22 close to the base of the cup-shaped portion 12a, compressing the bag 20 until its internal volume is reduced practically to zero, it is necessary for the screw 32 to have a length corresponding approximately to the distance separating the base of the cup-shaped portion 12a from the rear face of the motor 24 which face the plate 34 is intended to abut. Conversely, a similar distance has to be provided between the base of the shell portion 12b and the front face of the motor 24 close to which face the plate 22 is brought in its most retracted position, that is, the most extended condition of the bag 20.

This means that the casing 12 has to have an overall axial size corresponding in practice to the sum of the axial thickness of the motor 24 and approximately twice the length of the stroke of the plate 22.

The object of the present invention is to provide a solution which overcomes this intrinsic constraint so as correspondingly to reduce the dimensions of a pumping element such as the device 10 shown in Figure 1.

According to the present invention, this object is achieved by virtue of an actuator for pumping elements having the specific characteristics recited in the characterising portion of claim 1.

The invention will now be described, purely by way of non-limiting example, with reference to the appended drawings, in which:
Figure 1, which relates to the prior art, has already been described in detail in the introductory part of the present description,
Figure 2 shows an actuator formed according to the invention from a viewpoint essentially corresponding to that of Figure 1, and
Figures 3 and 4 show the element indicated by the arrow III in Figure 2 in two different operating positions.

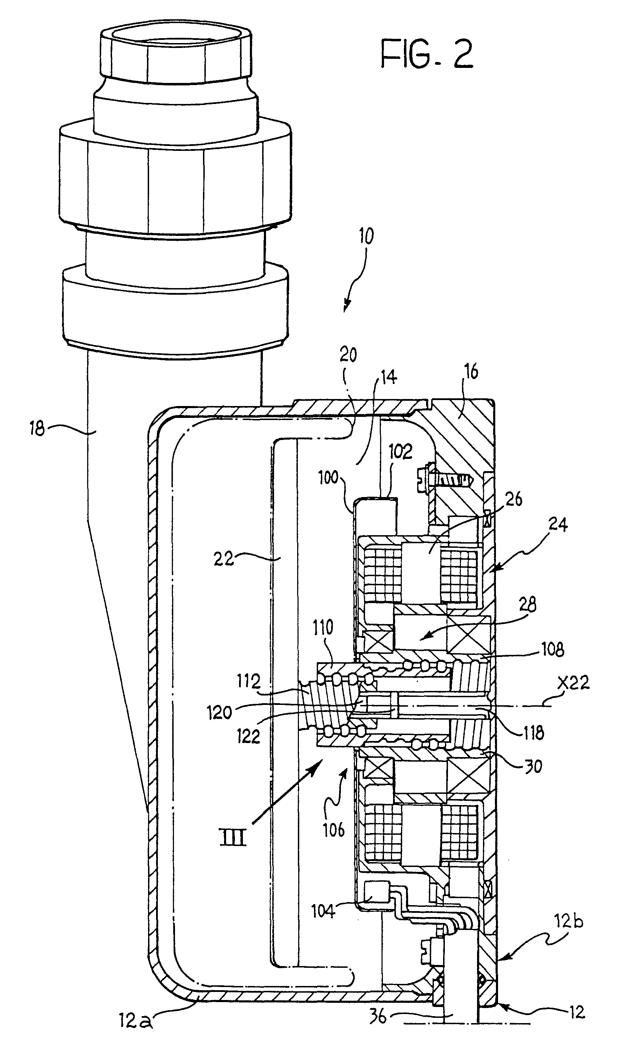

With reference to Figure 2, it should be noted that parts and components identical or functionally equivalent to those already described with reference to Figure 1 are indicated by the same reference numerals used above. This applies in particular to the motor 24 which, in the embodiment shown in Figure 2, has a stator 26 with radial dimensions which are further reduced in comparison with those of the motor shown in Figure 1.

A rotor element, indicated 100 in Figure 2, is keyed to the rotor 28 of the motor 24 and has a skirt portion 102 (having locally differentiated surface characteristics, for example, owing to the presence of notches) which extends around the periphery of the stator 26 forming an annular space in which electro-optical sensors (detector units) 104 are located. This is in order to form (according to a known solution) a so-called optical encoder the function of which is to detect the rotation parameters of the motor 24 (in particular, the position reached by the motor 24 during rotation) so as to be able to obtain an incremental position reference during the detection of the movement of the plate 22.

An important characteristic of the solution according to the invention is that the screw 32 is replaced by a telescopic element constituted by a telescopic screw 106 also preferably constructed in the form of a ball-bearing screw.

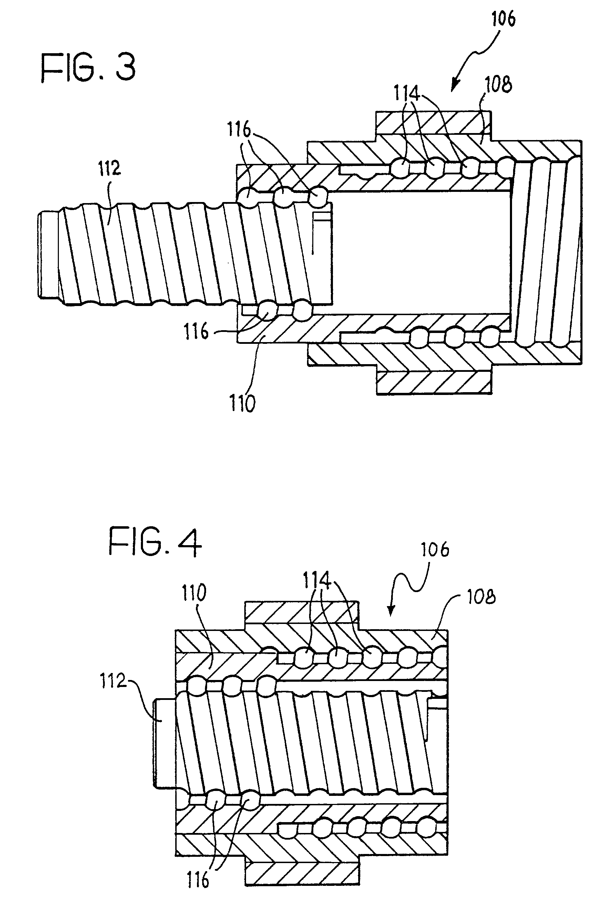

As can best be seen in the views of Figures 3 and 4 which show the screw 106 in its most axially extended condition (Figure 3) and in its most axially contracted condition (Figure 4), the screw 106 is composed essentially of three elements, that is:
- a cylindrical lead screw 108 for fitting (usually by interference) inside the rotor 28 of the motor 24 so as to be fixed for rotation therewith,
- an intermediate cylindrical sleeve 110 the outer surface of which has the characteristics of a screw engaged with the lead screw 108 and the inner surface of which has the characteristics of a further lead screw of smaller diameter than the lead screw 108, and
- an inner element 112, the outer surface of which has the characteristics of a screw engaged with the internal thread (lead screw) of the sleeve 110; the inner element 112 is intended to support the plate 22 in a central position.

As already stated, both the coupling between the lead screw 108 and the sleeve 110 and the coupling between the latter and the inner element 112 are achieved according to the typical configuration of a ball-bearing screw.

The respective ball races (some of which are indicated 114 and 116) cannot readily be appreciated from Figures 3 and 4. However, this is a solution widely known in the art which does not need to be described in greater detail herein.

The various threads provided on the elements 108, 110 and 112 are preferably formed in a manner such that, although the respective couplings (lead screw 108/sleeve 110; sleeve 110/inner element 112) have different diameters, they have uniform and identical pitches, thus giving rise to uniform distances of axial translation of the plate 22 for a given angular travel of the rotor 28.

It will also be appreciated that the solution shown in the appended drawings may be generalized so as to give rise to a telescopic screw element having a larger number of elements, for example, by transmitting the movement from the lead screw 108 to the inner element 112 by means of more than one intermediate sleeve element such as the element 110 shown in the drawings. The solution shown by way of example herein has, however, been found in experiments carried out up to now by the Applicant to be an ideal combination of operating requirements with the need to limit the complexity of the solution adopted.

Moreover, the restraint of the plate 22 against rotation which may be induced by the motor 24 which, in the device of Figure 1, is achieved by means of one or more rods 38, is achieved in the device of Figure 2 by the provision of a pin 118 extending from the shell portion 12b towards the interior of the shell 12 in alignment with the axis X22 and having (directly or as a result of the location thereon of a tubular insert 120 restrained against rotation by a pin 122) a prismatic cross-section complementary with the cross-section of an axial hole formed in the inner element 112 of the screw 106.

Finally, the function of the plate 34 of Figure 1 as an absolute reference for the position of the plate in abutment with the body of the motor 24, is performed in the device of Figure 2 by suitable radial abutment teeth (not shown) which, with reference to Figure 3, prevent complete unscrewing of the element 112 from the element 110 and of the element 110 from the element 108.

As shown in Figure 2, the solution according to the invention operates in essentially the same way as described above with reference to the solution of the prior art shown in Figure 1.

In this case the cyclic pumping of the blood by the device 10 is also achieved by a reciprocating translational movement of the plate 22 along the axis X22. This movement is brought about by the rotation of the motor 24 alternately in the two senses. However, in this case, the rotary movement of the motor 24 and the resulting translational movement of the plate 22 are also associated with a cyclic contraction and extension of the screw 106 owing to its telescopic structure. In particular, the most retracted position of the plate 22 in which it is close to the motor 24 corresponds to the arrangement of the screw 106 in the most axially contracted condition shown in Figure 4; in this situation, the inner element 112 is completely, or almost completely, housed in the sleeve 110 which in turn is almost completely housed in the lead screw 108, the axial dimensions of which are determined so as to correspond essentially to the axial thickness of the motor 24.

When this condition, which corresponds to the maximum possible extension permitted for the bag 20, has been reached, a change of the sense of rotation of the motor 24 brings about:
- on the one hand, axial translation of the sleeve 110 relative to the lead screw 108 with a consequent forward movement towards the extended position shown in Figure 3, and
- on the other hand, axial translation of the inner element 112 relative to the sleeve 110 with a consequent completion of the extension movement of the screw.

It is not envisaged that the aforesaid forward movements should usually necessarily take place sequentially or in a manner established beforehand (meaning one and then the other or at least partially simultaneously). As a general rule, these movements take place in an undifferentiated manner in the sense that the succession and/or overlapping of the relative axial translational movements between the sleeve 110 and the lead screw 108, on the one hand, and between the element 112 and the sleeve 110, on the other hand, is established by the specific conditions of friction, of local stress, etc. and may even vary from device to device, the final result (that is, the movement of the screw 106 from the axially contracted position of Figure 4 to the axially extended position of Figure 3) remaining the same. Naturally solutions are also possible in which a predetermined order is imposed on the carrying-out of the relative movements of the elements making up the screw 106 by various means (for example, by alteration of the pitches of the threads, etc).

As already stated above, the solution according to the invention gives preference to the use of a uniform and identical pitch (the distance of axial translation per unit of rotation) for all of the various elements. This solution has the advantage of establishing a precise and uniform relationship between the angular movement of the motor 24 and the axial translation imparted to the plate 22 and hence the compression of the bag 20.

When the plate 22 has been advanced towards the position in which the bag 20 is most squashed, the plate 22 can be returned backwards towards the motor 24 by a change of the sense of rotation of the motor 24, returning the screw 116 to the contracted position shown in Figure 4.

It is clear from the foregoing and, in particular, from an observation of Figures 2 to 4, that the solution according to the invention considerably reduces the dimensions of the shell 12. This applies particularly to the axial direction since the rear face of the motor 24 (that is the face of the motor facing away from the plate 22) can, in fact, be brought close to the corresponding wall of the shell 12. In fact, in the embodiment shown in Figure 2, this wall of the shell 12 is in practice constituted by a face of the casing of the motor 24.

With regard to the overall axial length measured along the axis X22, it can be seen that, with the invention, it is reduced in practice to the sum of the axial thickness of the motor 24 and the stroke of the plate 22. In practice, the axial length of the actuating portion 16 can be reduced by more than half, giving rise to a considerable reduction in the dimensions of the device 10 as a whole and consequently facilitating implantation thereof, particularly when solutions which correspondingly reduce the axial length of the pumping section 14 are implemented.

As already stated, the telescopic screw described could include a number of elements other than the three elements 108, 110, 112 described above. Moreover, although this solution is considered less preferable for structural reasons, the mounting arrangement of the telescopic screw could be reversed in comparison with that described, for example, by the connection of the innermost element (the element 112 in the appended drawings) to the motor 24 and of the outer lead screw (108 in the drawings) to the plate 22.

Naturally, the principle of the invention remaining the same, the details of construction and forms of embodiment may be varied widely with respect to those described and illustrated, without thereby departing from the scope of the present invention, particularly as regards its possible application to pumping elements in general without being expressly limited to the field of pumping devices for medical use such as cardiac assistance devices.

## Claims

1. An actuator for pumping elements comprising a contractible pumping element in the form of a flexible bag (20) and a thrust member (22) which is movable with reciprocating motion and is adapted to act on the flexible bag (20) in order to bring about the contraction, the actuating section (16) comprising:
- a rotary motor (24) having a rotor (28) rotatable about a respective axis (X22), and
- a screw element (106) which is coupled to the rotor (28) and to the thrust member (22) in order to convert the rotation of the rotor (28) into translational movement of the thrust member (22), wherein the screw element is constituted by a telescopic element (106) which, as a result of the rotation of the rotor (28), can change between an axially contracted condition (Figure 4) and an axially extended condition (Figure 3),
**characterized in that** said telescopic element (106) comprises:
- a lead screw (108),
- at least one sleeve element (110) which is engaged externally with the lead screw (108) and in turn is configured internally as a further respective lead screw, and
- an inner element (112) engaged with the further lead screw.

2. An actuator according to Claim 1, **characterized in that** the screw (106) is a ball-bearing screw.

3. An actuator according to Claim 1, **characterized in that** the lead screw (108) is connected to the rotor (28) so as to be rotated by the rotor (28).

4. An actuator according to any one of the preceding claims, **characterized in that** the telescopic element (106) comprises a plurality, of screw couplings (108, 110); 110, 112) with different diameters and uniform pitch in order to achieve uniform distances of axial translation of the thrust element (22) for a given angular travel of the rotor (28).

5. An actuator according to any one of the preceding claims, **characterized in that** the motor (24) has a generally disc-like shape, the rotor (28) defining a central orifice of the disk-like shape in which the telescopic screw (106) is fitted.

6. An actuator according to Claim 5, **characterized in that**, in the axially contracted position, the telescopic screw (106) has an axial length corresponding approximately to the axial thickness of the motor (24).

7. An actuator according to any one of the preceding claims, **characterized in that** the motor (24) is housed in a respective portion of a shell-like casing (12) and has an end face which faces away from the thrust element (22) and is located in close proximity to a respective end face of the shell (12b).

8. An actuator according to any one of the preceding claims, **characterized in that** the motor (24) comprises a stator (26) with an associated prismatic rod (110, 120) which, in order to prevent the thrust element (22) from being rotated as a result of the rotation of the motor (24) cooperates, in conditions of free relative axial movement and resistance to rotation, with the part (112) of the telescopic element (106) which cooperates with the thrust element (22).

9. An actuator according to Claim 8, **characterized in that** the prismatic rod (118, 120) cooperates with the inner element (112) of the telescopic screw (106).

10. An actuator according to any one of the preceding claims, **characterized in that** the rotor (28) of the motor (24) is associated with an optical encoder (100, 102, 104) which can provide an indication of the position reached by the motor (24).

11. An actuator according to Claim 10, **characterized in that** the optical encoder comprises a detector unit (104) associated with the stator (26) of the motor (24) as well as a disc-like element (100) rotated by the rotor (28) of the motor (24) and having at least one portion (102) which has locally differentiated surface characteristics, and cooperates with the detector unit (104).

## Patentansprüche

1. Ein Antrieb für Pumpelemente mit einem kontrahierbaren Pumpelement in der Form einer flexiblen Tasche (20) und einem Stoßelement (22), welches hin- und herbewegbar und geeignet ist, auf die flexible Tasche (20) zu wirken, um die Kontrahierung zu bewirken, wobei das Betätigungssytem aufweist:
- einen Drehmotor (24), welcher einen Rotor (28) aufweist, der um eine entsprechende Achse (X22) rotierbar ist, und
- ein Schraubenelement (106), das mit dem Rotor (28) und dem Stoßelement (22) verbunden ist, um die Rotation des Rotors (28) in eine Translationsbewegung des Stoßelements (22) umzuwandeln, wobei das Schraubenelement in der Form eines Teleskopelements (106) ausgebildet ist, welches aufgrund der Rotation des Rotors (28) in der Lage ist, zwischen einer axial kontrahierten Position (Figur 4) und einer axial ausgedehnten Position (Figur 3) zu wechseln, **dadurch gekennzeichnet, daß** das genannte Teleskopelement (106) umfaßt:
- eine Verstellschraubenspindel (108),
- zumindest ein Hülsenelement (110), welches außen mit der Verstellschraubenspindel (108) in Eingriff steht und wiederum im Inneren als eine weitere Verstellschraubenspindel ausgebildet ist, und
- ein inneres Element (112), welches mit der weiteren Verstellschraubenspindel in Eingriff steht.

2. Ein Antrieb nach Anspruch 1, **dadurch gekennzeichnet, daß** die Schraube (106) eine Kugellaufschraube ist.

3. Ein Antrieb nach Anspruch 1, **dadurch gekennzeichnet, daß** die Schraube (108) mit dem Rotor (28) verbunden ist, so daß diese mit dem Rotor (28) rotiert.

4. Ein Antrieb nach einem der vorherigen Ansprüche, **dadurch gekennzeichnet, daß** das Teleskopelement (106) eine Vielzahl von Verschraubungen (108, 110; 110, 112) mit unterschiedlichen Durchmessern und einheitlichen Steigungen aufweist, um einheitliche Strecken der axialen Translation des Stoßelements (22) für einen gegebenen Winkelweg des Rotors (28) zu erreichen.

5. Ein Antrieb nach einem der vorhergehenden Ansprüche, **dadurch gekennzeichnet, daß** der Motor (24) allgemein eine scheibenförmige Form aufweist, und der Rotor (28) eine zentrale Öffnung dieser scheibenförmigen Form definiert, in welche die Teleskopschraube (106) eingesetzt ist.

6. Ein Antrieb nach Anspruch 5, **dadurch gekennzeichnet, daß** die Teleskopschraube (106) in der axial kontrahierten Position eine axiale Länge aufweist, welche ungefähr der axialen Dicke des Motors (24) entspricht.

7. Ein Antrieb nach einem der vorhergehenden Ansprüche, **dadurch gekennzeichnet, daß** der Motor (24) in einem entsprechenden Teil eines kastenartigen Gehäuses (12) untergebracht ist und eine Endfläche aufweist, welche von dem Stoßelement (22) weggerichtet ist und sich in großer Nähe zu einer entsprechenden Endfläche des Gehäuses (12b) angeordnet ist.

8. Ein Antrieb gemäß einem der vorhergehenden Ansprüche, **dadurch gekennzeichnet, daß** der Motor (24) einen Stator (26) mit einer zugehörigen prismatischen Stange (110, 120) aufweist, welche, um zu verhindern, daß das Stoßelement (22) als Resultat der Rotation des Motors (24) in Rotation versetzt wird, unter Bedingungen einer freien relativen axialen Bewegung und Widerstand gegen Rotation mit dem Teil (112) des Teleskopelements (106) kooperiert, welches mit dem Stoßelement (22) kooperiert.

9. Ein Antrieb nach Anspruch 8, **dadurch gekennzeichnet, daß** die prismatische Stange (118, 120) mit dem inneren Element (112) der Teleskopschraube (106) kooperiert.

10. Ein Antrieb nach einem der vorhergehenden Ansprüche, **dadurch gekennzeichnet, daß** der Rotor (28) des Motors (24) mit einem optischen Codierer (100, 102, 104) versehen ist, welcher in der Lage ist, eine Anzeige bezüglich einer momentanen Arbeitsposition des Motors (24) auszugeben.

11. Ein Antrieb nach Anspruch 10, **dadurch gekennzeichnet, daß** der optische Codierer eine Detektoreinheit (104), welche dem Stator (26) des Motors zugeordnet ist, sowie ein scheibenartiges Element (110), das durch den Rotor (28) des Motors (24) in Rotation versetzt wird und wenigstens einen Bereich (102), welcher lokal verschiedene Oberflächeneigenschaften aufweist und mit der Detektoreinheit (104) kooperiert, aufweist.

## Revendications

1. Actionneur pour des éléments de pompage comprenant un élément de pompage capable de se contracter sous la forme d'un sac flexible (20) et un élément poussoir (22) qui est mobile avec un mouvement en va-et-vient et qui est adapté pour agir sur le sac flexible (20) afin d'exécuter la contraction, la partie d'actionnement (16) comprenant :
- un moteur rotatif (24) ayant un rotor (28) en rotation autour d'un axe rotatif (X22), et
- un élément à vis (106) qui est accouplé au rotor (28) et à l'élément poussoir (22) afin de convertir la rotation du rotor (28) en un mouvement de translation de l'élément poussoir (22) ; dans lequel ledit élément à vis est constitué par un élément télescopique (106) qui, en résultat de la rotation du rotor (28) peut changer antre une condition axialement contractée (figure 4) et une condition axialement déployée (figure 3),
**caractérisé en ce que** ledit élément télescopique (106) comprend :
- une tige filetée (108),
- au moins un élément en fourreau (110) qui est engagé à l'extérieur avec la tige filetée (108) et qui est configuré à son tour à l'intérieur comme une autre tige filetée respective, et
- un élément intérieur (112) engagé avec ladite autre tige filetée.

2. Actionneur selon la revendication 1, **caractérisé en ce que** ledit élément à vis (106) est une vis à circulation de billes.

3. Actionneur selon la revendication, **caractérisé en ce que** la tige filetée (108) est connectée au rotor (28) de manière à être mise en rotation par le rotor (28).

4. Actionneur selon l'une quelconque des revendications précédentes,
**caractérisé en ce que** l'élément télescopique (106) comprend une pluralité d'accouplements à vis (108, 110 ; 110, 112) avec des diamètres différents et un pas uniforme afin d'obtenir des distances uniformes pour la translation axiale de l'élément poussoir (22) pour un déplacement angulaire donné du rotor (28).

5. Actionneur selon l'une quelconque des revendications précédentes. **caractérisé en ce que** le moteur (24) a généralement une forme analogue à un disque, le rotor (28) définissant un orifice central de la forme analogue à un disque, dans lequel l'élément à vis télescopique (106) est logé.

6. Actionneur selon la revendication 5, **caractérisé en ce que**, dans la position axialement contractée, la vis télescopique (106) a une longueur axiale qui correspond approximativement à l'épaisseur axiale du moteur (24).

7. Actionneur selon l'une quelconque des revendications précédentes, **caractérisé en ce que** le moteur (24) est abrité dans une partie respective d'un boîtier (12) en forme de coque, et **en ce qu'**il comporte une face terminale détournée de l'élément poussoir (22) et située à proximité étroite d'une face terminale respective de la coque (12b).

8. Actionneur selon l'une quelconque des revendications précédentes, **caractérisé en ce que** le moteur (24) comprend un stator (26) avec une barre prismatique associée (110, 120) qui, afin d'empêcher à l'élément poussoir (22) d'être mis en rotation en résultat de la rotation du moteur au (24) et qui, dans des conditions de mouvement axial relatif est libre et de résistance à la rotation, coopère avec la partie (112) de l'élément télescopique (106) qui coopère avec l'élément poussoir (22).

9. Actionneur selon la revendication 8, a été usé en ce que la barre prismatique (118, 120) coopère avec l'élément intérieur (112) de la vis télescopique (106).

10. Actionneur selon l'une quelconque des revendications précédentes, **caractérisé en ce que** le rotor (28) du moteur (24) est associé à un codeur optique (100, 102, 104) qui est en mesure de fournir une indication de la position atteinte par le moteur (24).

11. Actionneur selon la revendication 10, **caractérisé en ce que** le codeur optique comprend une unité de détection (104) associée au stator (26) du moteur (24), ainsi qu'un élément en forme de disque (10) mis en rotation par le rotor (28) du moteur (24) et comportant au moins une partie (102) qui présente des caractéristiques de surface avec des différences locales et qui coopère avec l'unité de détection (104).
